# FASCICULE DE BREVET EUROPEEN

(11) **EP 0 475 803 B1**
(45) Date de publication et mention de la délivrance du brevet: **03.05.1995**
(21) Numéro de dépôt: 91402228.0
(22) Date de dépôt: 12.08.1991
(51) Int. Cl.: G01N 21/57, A61B 5/103

(54) **Appareil destiné à permettre d'évaluer la brillance d'une surface, en particulier de la peau**
Vorrichtung zur Messung des Glanzvermögens einer Oberfläche, insbesondere der Haut
Apparatus to measure the shininess of a surface, particularly of the skin

(30) Priorité: 16.08.1990 FR 9010379
(43) Date de publication de la demande: 18.03.1992
(73) Titulaire: L'OREAL, 75008 Paris (FR)
(72) Inventeur: Bazin, Roland, F-94400 Vitry/Seine (FR); Chommeloux, Luc, F-78000 Versailles (FR); Obadia, Gérard, F-92120 Montrouge (FR); Chardron, Hervé, F-91160 Longjumeau (FR)
(74) Mandataire: Peuscet, Jacques

(56) Documents cités:
- EP-A- 0 198 759
- EP-A- 0 335 163
- FR-A- 2 175 259
- FR-A- 2 499 717
- FR-A- 2 650 890
- US-A- 4 398 541
- PATENT ABSTRACTS OF JAPAN, vol. 13, no. 475 (P-950)(3823) 27 Octobre 1989 ; & JP-A-1 187 437

## Description

L'invention est relative à un appareil destiné à l'examen d'une surface, comprenant une source de lumière propre à envoyer un faisceau incident sur la surface à examiner, des moyens comprenant un polariseur et au moins un analyseur permettant d'apprécier la réflexion soit avec une orientation parallèle des directions du polariseur et de l'analyseur, soit avec une orientation à angle droit des susdites directions, le polariseur étant disposé entre la source de lumière et la surface, tandis que l'analyseur est disposé sur le trajet du faisceau réfléchi, des moyens photodétecteurs sensibles à la lumière renvoyée par la surface étant en outre prévus, la source de lumière étant directive et le faisceau incident polarisé tombant sur la surface à étudier selon un angle d'incidence compris entre 0° et 90° limites exclues, la direction de polarisation du faisceau incident étant perpendiculaire au plan d'incidence, l'appareil étant agencé pour mesurer la réflexion suivant au moins deux directions de réflexion différentes, l'une de ces directions de réflexion étant sensiblement symétrique de la direction incidente relativement à la normale à la surface.

L'invention concerne plus particulièrement, mais non exclusivement, un tel appareil pour évaluer la brillance de la peau.

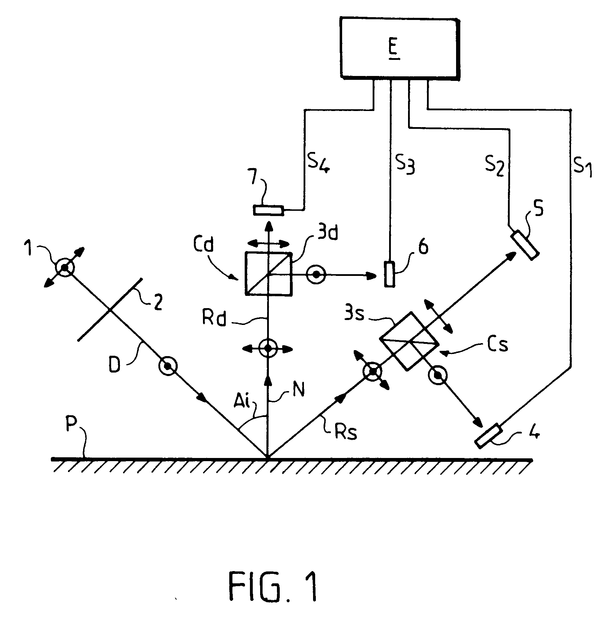

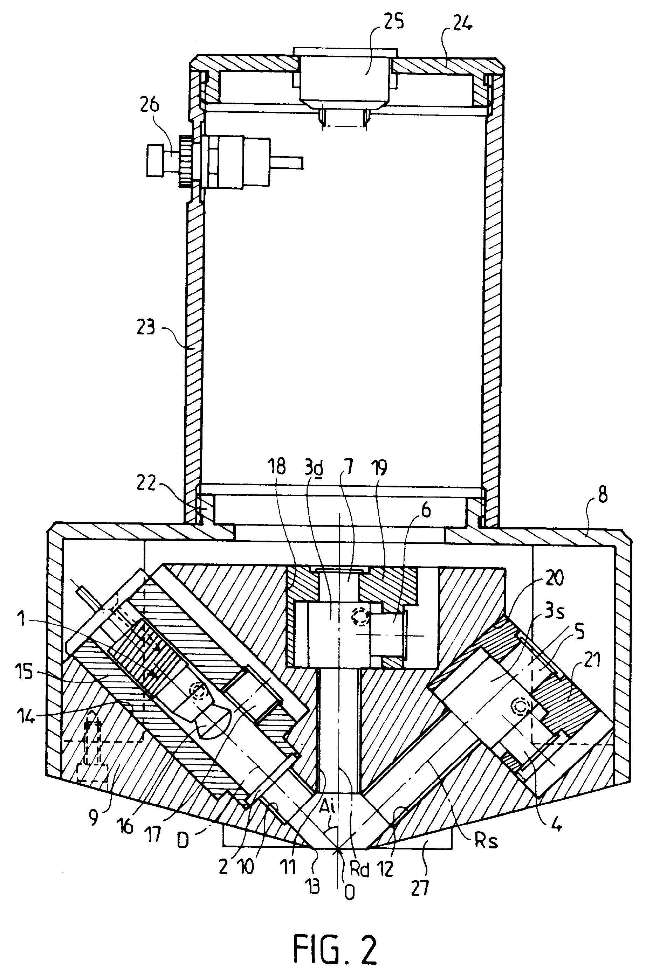

Un appareil de ce type est montré par EP-A-0 335 163 et, plus particulièrement, par la figure 4 de ce document, résumant l'art antérieur. L'appareil en question est destiné à détecter la présence de matières étrangères sur les surfaces d'un substrat. Selon la réalisation de ladite figure 4, deux faisceaux réfléchis R1 et R2, suivant deux directions de réflexion différentes, sont pris en compte. Chaque faisceau réfléchi est analysé suivant un angle de polarisation, à l'aide d'un analyseur associé, sans qu'il y ait séparation de ce faisceau en deux parties. Cette solution de la figure 4 permet uniquement une distribution directionnelle non uniforme de la lumière réfléchie R et une variation de manière imprévisible de l'intensité des faisceaux R1 et R2. Pour remédier à ces inconvénients, il est proposé de travailler sur un seul faisceau Ro de lumière réfléchie diffuse, selon la réalisation des figures 1 et 2, et, sur cette seule direction de réflexion, on effectue la différence entre la réflexion avec des directions de polarisation et d'analyse parallèles (faisceau r1 de la figure 1) et la réflexion avec des directions de polarisation et d'analyse perpendiculaires (faisceau r2 de la figure 2). En conclusion, ce document EP-A-0 335 163 ne concerne pas une mesure de brillance mais et invite à travailler sur une seule direction de réflexion.

On connaît par ailleurs, d'après EP-A-0 198 759 un appareil destiné à permettre l'évaluation de la brillance d'une surface, en particulier de la peau. Cet appareil comprend une source de lumière propre à envoyer un faisceau incident sur la surface à examiner, ce faisceau étant réfléchi dans deux directions différentes ; des moyens photodétecteurs sensibles à la lumière réfléchie permettent d'effectuer une appréciation de la réflexion spéculaire suivant l'une des directions, et de la réflexion diffuse suivant l'autre des directions de réflexion. L'évaluation de la brillance est effectuée par différence entre les intensités des réflexions spéculaire et diffuse (voir notamment page 10 lignes 33 à 35). Une seule brillance "globale" est appréciée par cet appareil. Il n'est pas question de brillance de types différents, à savoir brillance spéculaire et brillance diffuse.

D'après FR-A-2 499 717, on connaît un procédé pour déterminer les défauts et les qualités d'une pièce de bois ; il n'est pas question de brillance spéculaire et de brillance diffuse.

La société déposante a en outre proposé dans une demande de brevet antérieure FR-A-2 650 890 (89 10 709), déposée le 9 août 1989, un appareil pour évaluer la brillance de la peau. Les essais effectués ont montré qu'un tel appareil, tout en donnant des résultats satisfaisants, avait une sensibilité et un pouvoir discriminateur relativement réduits.

L'invention a pour but, surtout, de fournir un appareil pour évaluer la brillance d'une surface, en particulier de la peau, qui soit plus sensible et plus discriminant et qui permette de différencier certains types de brillance par ses propriétés de directivité. Il est souhaitable, en outre, que l'appareil permette d'effectuer la mesure sur une zone réduite, presque ponctuelle, et en s'affranchissant de la couleur.

Selon l'invention, un appareil du type défini précédemment, est caractérisé par le fait qu'il comporte, pour permettre d'évaluer la brillance de la surface, en particulier de la peau, des moyens permettant d'effectuer, pour chaque direction de réflexion, la différence entre la réflexion avec des directions de polarisation et d'analyse parallèles et la réflexion avec des directions de polarisation et d'analyse perpendiculaires, les différences ainsi obtenues constituant une appréciation de la brillance dite spéculaire et de la brillance dite diffuse.

Avantageusement, l'angle d'incidence du faisceau polarisé sur la surface est d'environ 45° par rapport à la normale.

La deuxième direction de réflexion considérée est avantageusement située dans une plage de plus ou moins 10° de part et d'autre de la direction normale à la surface à étudier. Cette deuxième direction est, de préférence, sensiblement normale à ladite surface ; elle est généralement située dans le plan d'incidence.

Les moyens analyseurs comprennent, pour chaque direction de réflexion étudiée, un système permettant de séparer angulairement les signaux lumineux polarisés parallèlement et perpendiculairement au plan d'incidence ; avantageusement, deux photodétecteurs sont associés à chaque système séparateur pour permettre une mesure simultanée de ces signaux lumineux.

Chaque système séparateur est avantageusement constitué par un cube séparateur de polarisation.

Un tel appareil peut être utilisé pour étudier des phénomènes biologiques qui influent sur la brillance de la peau, par exemple la cinétique de la secrétion sébacée, ou pour étudier les conséquences sur la brillance de la peau de l'application de produits cosmétiques, notamment l'aptitude d'une base matifiante à diminuer la brillance cutanée provoquée par le sébum.

L'invention consiste, mises à part les dispositions exposées ci-dessus, en un certain nombre d'autres dispositions dont il sera plus explicitement question ci-après à propos d'un exemple de réalisation décrit en détail avec référence aux dessins ci-annexés, mais qui n'est nullement limitatif.

La figure 1, de ces dessins, est un schéma d'un appareil conforme à l'invention.

La figure 2, enfin, est une coupe verticale de l'appareil conforme au schéma de la figure 1.

En se reportant à la figure 1, on peut voir le schéma d'un appareil destiné à permettre d'évaluer la brillance d'une surface P, en particulier de la peau.

Cet appareil comprend une source 1 de lumière non polarisée. Sur le schéma, la lumière non polarisée est représentée symboliquement par un cercle avec un point au centre, traversé par une double flèche suivant un diamètre ; une lumière polarisée perpendiculairement au plan d'incidence est représentée par un cercle avec un point au centre, tandis qu'une lumière polarisée parallèlement au plan d'incidence est représenté uniquement par une double flèche.

La source de lumière 1 est une source de lumière blanche, directive et est équipée, par exemple, d'un système optique (non représenté) donnant un faisceau parallèle de direction D. Un polariseur 2 rectiligne est disposé avec son plan moyen orthogonal à la direction D, entre la source 1 et la surface P de sorte que la lumière tombant suivant la direction D sur la peau P est polarisée.

L'angle d'incidence Ai, formé entre la direction D du faisceau incident et la direction normale N à la surface P est compris entre 0° (incidence normale) et 90° (incidence rasante), ces limites étant exclues. De préférence, l'angle Ai est égal à 45°, comme représenté sur la figure 1, ou voisin de cette valeur.

La direction de polarisation du faisceau incident, après traversée du polariseur 2, est perpendiculaire au plan d'incidence.

L'appareil est prévu pour mesurer la réflexion suivant deux directions de réflexion Rs, Rd différentes. La direction Rs, qui correspond à la réflexion spéculaire, est symétrique de la direction D par rapport à la normale N. La lumière réfléchie suivant la direction Rd est obtenue par réflexion diffuse de la lumière qui a pénétré à l'intérieur de la peau P, ou bien par une réflexion de surface, si celle-ci présente une certaine irrégularité.

De préférence, la deuxième direction de réflexion Rd choisie pour effectuer les mesures est confondue avec la direction N, ou voisine de cette direction, à plus ou moins 10° près. Cette direction Rd est de préférence située dans le plan d'incidence.

La lumière réfléchie suivant les deux directions Rs et Rd n'est plus polarisée linéairement, contrairement à la lumière incidente.

Des moyens analyseurs Cs, Cd, sont prévus pour chaque direction de réflexion étudiée et comprennent un système séparateur, avantageusement constitué par un cube séparateur de polarisation 3s, 3d, du type prisme de Wollaston, qui permet de séparer angulairement, à angle droit dans l'exemple considéré, les signaux lumineux polarisés parallèlement et perpendiculairement au plan d'incidence, pour chaque faisceau réfléchi Rs, Rd.

Deux photodétecteurs respectivement 4, 5 et 6, 7 sont associés à chaque système séparateur pour permettre une mesure simultanée des signaux lumineux. Ces photodétecteurs sont reliés à des moyens électroniques E permettant de traiter les signaux pour fournir les résultats cherchés.

Le photodétecteur 4 reçoit la fraction du faisceau réfléchi Rs polarisée perpendiculairement au plan d'incidence et fournit un signal S1.

Le photodétecteur 5 reçoit la fraction du faisceau Rs polarisée parallèlement au plan d'incidence et fournit un signal S2.

Le photodétecteur 6 reçoit la fraction du faisceau Rd polarisée perpendiculairement au plan d'incidence et fournit un signal S3.

Enfin, le photodétecteur 7 reçoit la fraction du faisceau Rd polarisée parallèlement au plan d'incidence et fournit un signal S4.

Pour chaque direction de réflexion Rs, Rd, le faisceau comprend une composante d'intensité Ib, due essentiellement à la brillance de la surface, et une composante d'intensité Ic à l'origine de la couleur de la surface considérée, et provenant d'une partie de la lumière qui a pénétré dans le milieu où elle a subi des phénomènes de diffraction avant d'être renvoyée vers les photodétecteurs. En pratique on peut écrire que :
S1 = (Ib + 1/2 Ic)s,
S2 = (1/2 Ic)s, pour le faisceau Rs correspondant à la réflexion spéculaire.

Pour le faisceau Rd, correspondant à la réflexion diffuse, on peut écrire :
S3 = (Ib + 1/2 Ic)d
S4 = (1/2 Ic)d.

En faisant la différence entre les deux résultats S1 et S2, on obtient la valeur S1 - S2 = (Ib)s qui représente la mesure de la brillance dans la direction Rs, brillance que l'on peut appeler brillance spéculaire Bs.

En faisant la différence entre les résultats S3 et S4, on obtient l'expression S3 - S4 = (Ib)d qui représente la brillance dans la direction Rd, et que l'on peut appeler brillance diffuse Bd.

Les résultats S1, S2, S3 et S4 peuvent être affichés sur un écran par les moyens électroniques E et les différences peuvent être effectuées manuellement.

Selon une autre possibilité, les moyens électroniques E sont agencés pour effectuer directement la différence entre les signaux S1 et S2, d'une part, et les signaux S3 et S4, d'autre part, et pour afficher ces différences, c'est-à-dire pour afficher la brillance spéculaire Bs et la brillance diffuse Bd.

La capacité de l'appareil selon l'invention, à distinguer les deux types de brillance Bs et Bd est tout-à-fait intéressante. En effet, si la peau présente un relief marqué, la directivité de la brillance sera faible et la valeur de la brillance spéculaire Bs ne sera que sensiblement supérieure à la brillance diffuse Bd.

Par contre, si la surface de la peau est très lisse, notamment après traitement par un produit cosmétique, la brillance sera beaucoup plus directive et la valeur de la brillance spéculaire Bs sera nettement supérieure à la valeur de la brillance diffuse Bd.

En se reportant à la figure 2 on peut voir un exemple de réalisation d'un appareil conforme au schéma de la figure 1.

L'appareil comprend une embase 8 dans laquelle est fixé, de manière démontable, un noyau 9 où sont prévus trois logements 10, 11, 12 admettant respectivement comme axes les directions D, Rd et Rs. Les axes des logements concourent en un point O qui constitue le centre d'une ouverture elliptique 13 prévue à la partie inférieure du noyau et qui est appliquée sur la surface P à examiner.

Le logement 10 se prolonge, du côté opposé au point O, par une chambre 14 de plus fort diamètre dans laquelle est disposé un manchon 15 coaxial à la direction D. A l'intérieur du manchon est placée la source de lumière 1 comportant une ampoule électrique 16 munie, par exemple, d'une lentille pour former un faisceau directif. Le filtre polarisant rectiligne 2 est disposé à la partie inférieure du manchon 15, orthogonalement à l'axe du manchon. Un élément photodétecteur 17 est monté dans la paroi du manchon 15, au niveau de la zone d'émission de l'ampoule 16 pour mesurer l'intensité lumineuse moyenne de cette source. Cet élément 17 est relié à des moyens électroniques non représentés propres à moduler le résultat,des mesures des photodétecteurs 4-7 en fonction de l'intensité de la source lumineuse.

Le logement 11 est prolongé, du côté opposé au point O, par une chambre 18 dans laquelle est placé un bloc 19 où sont logés le cube séparateur de polarisation 3d et les photodétecteurs associés 6, 7.

De la même manière le logement 12 est prolongé, du côté opposé au point O, par une chambre cylindrique 20 dans laquelle est placé un bloc 21 où sont montés respectivement le cube séparateur 3s et les photodétecteurs 4, 5.

Il est à noter qu'en adoptant pour l'angle Ai la valeur de 45° ; pour Rs la direction symétrique relativement à la normale ; et pour Rd, la direction de la normale, on peut loger l'ensemble de la source de lumière, des cubes séparateurs et des photodétecteurs dans un volume minimal.

L'embase 8 comporte, dans sa partie centrale supérieure, une ouverture entourée par une collerette 22 sur laquelle est fixé, notamment par vissage, un élément tubulaire 23 fermé, à sa partie supérieure, par un couvercle plan 24. Au centre de ce couvercle est prévu un connecteur électrique 25 permettant d'assurer les liaisons électriques avec l'extérieur notamment pour transmettre les résultats fournis par les éléments photodétecteurs 4, 5, 6, 7 et 17 reliés à ce connecteur. Un bouton-poussoir 26 est monté dans la paroi de l'élément 23 ; ce bouton 26 permet de commander l'allumage de la source lumineuse 16 pendant un temps déterminé au cours duquel est effectué une mesure. L'extinction est commandée manuellement par l'intermédiaire du bouton-poussoir 26, ou automatiquement. Les diverses liaisons électriques n'ont pas été représentées à l'intérieur de l'embase 8 et de l'élément tubulaire 23 qui constitue également un moyen de prise manuelle et d'application de la tête de mesure formée par l'ensemble.

On peut remarquer que l'extrémité inférieure du noyau 9 est tronconique, sa petite base étant constituée par l'ouverture 13. Pour assurer un bon appui de la tête de mesure sur la surface P on peut prévoir une couronne 27 dont le bord inférieur est situé sensiblement dans le plan de l'ouverture 13.

L'utilisation de l'appareil de la figure 2 résulte immédiatement des explications qui précèdent.

L'opérateur saisit l' appareil par la partie cylindrique de l'élément tubulaire 23 et applique le point O sur le centre de la zone où la mesure doit être effectuée. Puis, en appuyant sur le bouton-poussoir 26, l'opérateur déclenche la mesure en ce point.

L'appareil de l'invention, qui permet de quantifier dans l'espace les phénomènes de brillance dans deux directions différentes est particulièrement intéressant pour les mesures de brillance effectuées sur la peau, qui présente des irrégularités de surface. La lumière réfléchie par la surface de la peau peut être diffusée dans diverses directions et non pas uniquement dans la direction spéculaire, où elle reste maximale. L'appareil de l'invention tient compte de ce phénomène.

On peut dire que la brillance de la peau est la résultante de la brillance intrinsèque de la peau et de son état de surface.

Il est à noter que grâce aux cubes séparateurs de polarisation 3s, 3d, aucun mouvement mécanique n'est à effectuer entre le polariseur et l'analyseur, ce qui améliore la précision de l'appareil et permet d'effectuer une mesure simultanée des signaux polarisés parallèlement et perpendiculairement au plan d'incidence.

## Revendications

1. Appareil destiné à l'examen d'une surface, comprenant une source de lumière (1) propre à envoyer un faisceau incident sur la surface (P) à examiner, des moyens comprenant un polariseur (2) et au moins un analyseur (Cs, Cd) permettant d'apprécier la réflexion soit avec une orientation parallèle des directions du polariseur et de l'analyseur, soit avec une orientation à angle droit des susdites directions, le polariseur (2) étant disposé entre la source de lumière (1) et la surface (P), tandis que l'analyseur (Cs, Cd) est disposé sur le trajet du faisceau réfléchi, des moyens photodétecteurs (4, 5, 6, 7) sensibles à la lumière renvoyée par la surface (P) étant en outre prévus, la source de lumière (1) étant directive et le faisceau incident polarisé tombant sur la surface à étudier selon un angle d'incidence (Ai) compris entre 0° et 90° limites exclues, la direction de polarisation du faisceau incident étant perpendiculaire au plan d'incidence, l'appareil étant agencé pour mesurer la réflexion suivant au moins deux directions de réflexion différentes (Rs, Rd), l'une (Rs) de ces directions de réflexion étant sensiblement symétrique de la direction incidente (D) relativement à la normale (N) à la surface, caractérisé par le fait qu'il comporte, pour permettre l'évaluation de la brillance de la surface, en particulier de la peau, des moyens (Cd, Cs ; 4, 5, 6, 7 ; E) permettant d'effectuer, pour chaque direction de réflexion, la différence [(S1-S2) ; (S3-S4)] entre la réflexion avec des directions de polarisation et d'analyse parallèles et la réflexion avec des directions de polarisation et d'analyse perpendiculaires, les différences [(S1-S2) ; (S3-S4)] ainsi obtenues constituant une appréciation de la brillance dite spéculaire et de la brillance dite diffuse.

2. Appareil selon la revendication 1, caractérisé par le fait que l'angle d'incidence (Ai) du faisceau incident est d'environ 45° par rapport à la normale, l'angle de la première direction (Rs) de réflexion étant également voisin de 45°.

3. Appareil selon la revendication 1 ou 2, caractérisé par le fait que la deuxième direction de réflexion (Rd) est située dans une plage de plus ou moins 10° de part et d'autre de la direction (N) normale à la surface à étudier.

4. Appareil selon la revendication 3, caractérisé par le fait que la deuxième direction de réflexion (Rd) est sensiblement normale à ladite surface.

5. Appareil selon l'une des revendications précédentes, caractérisé par le fait que les moyens analyseurs comprennent, pour chaque direction de réflexion étudiée (Rs, Rd), un système (Cs, Cd) permettant de séparer angulairement les signaux lumineux polarisés parallèlement et perpendiculairement au plan d'incidence.

6. Appareil selon la revendication 5, caractérisé par le fait que deux photodétecteurs (4, 5 ; 6, 7) sont associés à chaque système séparateur (Cs, Cd), pour permettre une mesure simultanée des signaux lumineux.

7. Appareil selon la revendication 5 ou 6, caractérisé par le fait que chaque système séparateur (Cs, Cd) est constitué par un cube séparateur de polarisation (3s, 3d).

8. Application d'un appareil selon l'une quelconque des revendications précédentes à l'étude de la cinétique de la sécrétion sébacée.

9. Application d'un appareil selon l'une quelconque des revendications 1 à 7 à l'étude de l'aptitude pour une base matifiante à diminuer la brillance de la peau due au sébum.

## Patentansprüche

1. Vorrichtung zur Untersuchung einer Oberfläche mit einer Lichtquelle (1), die einen auf die zu untersuchende Oberfläche (P) einfallenden Lichtstrahl aussendet, Mitteln, die einen Polarisator (2) und wenigstens einen Analysator (Cs,Cd) umfassen, mit denen die Reflexion sowohl bei paralleler Ausrichtung von Polarisator und Analysator als auch bei rechtwinkliger Ausrichtung ermittelt werden kann, wobei der Polarisator (2) zwischen der Lichtquelle (1) und der Oberfläche (P) angeordnet ist, während der Analysator (Cs,Cd) auf dem Weg des reflektierten Strahls angeordnet ist, wobei außerdem für das von der Oberfläche (P) zurückgeworfene Licht empfindliche Photoempfänger (4,5,6,7) vorgesehen sind, wobei die Lichtquelle (1) gerichtet ist und der einfallende, polarisierte Strahl unter einem Einfallswinkel (Ai) zwischen 0° und 90°, Grenzen ausgeschlossen, auf die zu untersuchende Oberfläche trifft, wobei die Polarisationsrichtung des einfallenden Strahls senkrecht zur Einfallsebene ist, wobei die Vorrichtung zur Messung der Reflexion in mindestens zwei unterschiedlichen Reflexionsrichtungen (Rs,Rd) eingerichtet ist, wobei eine (Rs) dieser Reflexionsrichtungen im wesentlichen symmetrisch zur Einfallsrichtung (D) in Bezug auf die Oberflächennormale (N) ist, dadurch gekennzeichnet, daß sie zur Bestimmung des Glanzes der Oberfläche, insbesondere der Haut, Mittel (Cd,Cs;4,5,6,7;E) aufweist, die für jede Reflexionsrichtung die Bestimmung der Differenz [(S1-S2); (S3-S4)] zwischen der Reflexion bei paralleler Polarisations- und Analysierrichtung und der Reflexion bei zueinander senkrechter Polarisations- und Analysierrichtung ermöglicht, wobei die so ermittelten Differenzen [(S1-S2); (S3-S4)] ein Maß sowohl für den spiegelnden als auch für den diffusen Glanz bilden.

2. Vorrichtung gemäß Anspruch 1, dadurch gekennzeichnet, daß der Einfallswinkel (Ai) des einfallenden Strahls etwa 45°, bezogen auf die Normale, beträgt, wobei der Winkel der ersten Reflexionsrichtung (Rs) ebenfalls etwa 45° beträgt.

3. Vorrichtung gemäß Anspruch 1 oder 2, dadurch gekennzeichnet, daß die zweite Reflexionsrichtung (Rd) in einem Bereich von etwa 10° um die Normalrichtung der zu untersuchenden Oberfläche angeordnet ist.

4. Vorrichtung gemäß Anspruch 3, dadurch gekennzeichnet, daß die zweite Reflexionsrichtung (Rd) im wesentlichen senkrecht zur Oberfläche ist.

5. Vorrichtung gemäß einem der vorherigen Ansprüche, dadurch gekennzeichnet, daß die Analysatormittel für jede untersuchte Reflexionsrichtung (Rs,Rd) ein Bauteil (Cs,Cd) umfassen, das die Winkelauftrennung der parallel und senkrecht zur Einfallsebene polarisierten Lichtsignale ermöglicht.

6. Vorrichtung gemäß Anspruch 5, dadurch gekennzeichnet, daß zwei Lichtempfänger (4,5;6,7) jedem Trennbauteil (Cs,Cd) zugeordnet sind, damit die Lichtsignale gleichzeitig meßbar sind.

7. Vorrichtung gemäß Anspruch 5 oder 6, dadurch gekennzeichnet, daß jedes Trennbauteil (Cs,Cd) aus einem Polarisationsstrahlteiler-Würfel (3s,3d) besteht.

8. Verwendung einer Vorrichtung gemäß einem der vorherigen Ansprüche zur Untersuchung der Kinetik der Sebumabsonderung.

9. Verwendung einer Vorrichtung gemäß einem der Ansprüche 1 bis 7 zur Untersuchung der Eignung eines mattierenden Grundstoffes zur Verringerung des durch Sebum hervorgerufenen Glanzes der Haut.

## Claims

1. An apparatus intended for investigating a surface, comprising a light source (1) capable of passing an incident beam to the surface (P) to be investigated, means comprising a polariser (2) and at least one analyser (Cs, Cd) allowing the reflection to be assessed, either with a parallel orientation of the direction of the polariser and of the analyser, or with an orientation at right angles of the above mentioned directions, the polariser (2) being disposed between the light source (1) and the surface (P), whilst the analyzer (Cs, Cd) is disposed in the path of the reflected beam, provision being moreover made for photodetector means (4, 5, 6, 7) that are sensitive to the light reflected by the surface (P), the light source (1) being directional, and the polarised incident beam striking the surface to be investigated along an angle of incidence (Ai) comprised between 0° and 90° not including the limiting values, the direction of polarisation of the incident beam being perpendicular to the plane of incidence, the apparatus being arranged to measure the reflection along at least two different reflection directions (Rs, Rd), one (Rs) of these reflection directions being substantially symmetrical with the incident direction (D) relative to the normal (N) to the surface, characterized in that it includes, to allow the shine of the surface, in particular of the skin, to be assessed, means (Cd, Cs; 4, 5, 6, 7; E) making it possible to obtain for each reflection direction, the difference [(S1-S2) ; (S3-S4)] between the reflection with parallel directions of polarisation and of analysis, and the reflection with perpendicular directions of polarisation and of analysis, the differences [S1-S2) ; (S3-S4)] thus obtained constituting an assessment of the luminance termed specular luminance and the luminance termed diffuse luminance.

2. An apparatus according to claim 1, characterized in that the angle of incidence (Ai) of the incident beam is approximately 45° relative to the normal, the angle of the first reflection direction (Rs) also being close to 45°.

3. An apparatus according to claim 1 or 2, characterized in that the second reflection direction (Rd) is situated within a range of plus or minus 10° on either side of the direction (N) normal to the surface to be investigated.

4. An apparatus according to claim 3, characterized in that the second reflection direction (Rd) is substantially normal to the said surface.

5. An apparatus according to one of the preceding claims, characterized in that the analyser means comprise for each investigated reflection direction (Rs, Rd) a system (Cs, Cd) permitting an angular separation of the luminous signals polarised in parallel and perpendicularly to the plane of incidence.

6. An apparatus according to claim 5, characterized in that two photodetectors (4, 5, 6, 7) are associated with each separating system (Cs, Cd), to permit a simultaneous measurement of the luminous signals.

7. An apparatus according to claim 5 or 6, characterized in that each separating system (Cs, Cd) is constituted by a polarisation separator cube (3s, 3d).

8. Application of an apparatus according to any one of the preceding claims to an investigation of the kinetics of the sebaceous secretion.

9. Application of an apparatus according to any one of claims 1 to 7 to an investigation of the capability of a base to obtain a matt appearance to reduce shininess of the skin due to the sebum.
